(19) Europäisches Patentamt
European Patent Office
Office européen des brevets

(11) Veröffentlichungsnummer: **0 004 261**
**B1**

(12) **EUROPÄISCHE PATENTSCHRIFT**

(45) Veröffentlichungstag der Patentschrift: **28.04.82**

(21) Anmeldenummer: **78101547.4**

(22) Anmeldetag: **04.12.78**

(51) Int. Cl.³: **C 07 H 19/04,**
A 61 K 31/70 //C07F7/10,
C07D233/90

(54) **Neue Imidazolderivate, Verfahren zu deren Herstellung und Arzneimittel diese enthaltend.**

(30) Priorität: **01.02.78 CH 1095/78**

(43) Veröffentlichungstag der Anmeldung:
**03.10.79 Patentblatt 79/20**

(45) Bekanntmachung des Hinweises auf die Patenterteilung:
**28.04.82 Patentblatt 82/17**

(84) Benannte Vertragsstaaten:
**BE CH DE FR GB NL SE**

(56) Entgegenhaltungen:
**DE - A - 2 529 533**
**DE - A - 2 735 458**
**FR - A - 2 152 507**

Chem. Ber. 107, 892 (1974)

Helv. Chim. Acta, 47, 1171 (1959)

JOURNAL OF HETEROCYCLIC CHEMISTRY,
Vol. 15, Nr. 1 Januar 1978

(73) Patentinhaber: **F. HOFFMANN-LA ROCHE & CO. Aktiengesellschaft**
**CH-4002 Basel (CH)**

(72) Erfinder: **Wyss, Pierre-Charles, Dr.**
**Unterwartweg 27**
**CH-4132 Muttenz (CH)**

(74) Vertreter: **Kellenberger, Marcus, Dr. et al,**
**Grenzacherstrasse 124 Postfach 3255**
**CH-4002 Basel (CH)**

**EP 0 004 261 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue Imidazolderivate, Verfahren zu deren Herstellung und Arzneimittel diese enthaltend

Die vorliegende Erfindung betrifft neue Imidazolderivate, und zwar Ribofuranosyl-imidazolderivate der allgemeinen Formel

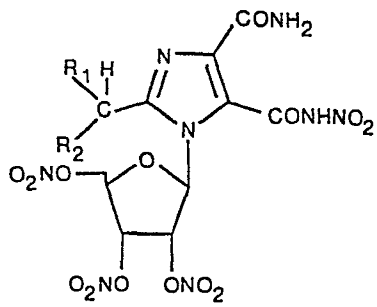

I

worin $R_1$ und $R_2$ je Wasserstoff oder Methyl bedeuten.

In der FR—A 2.152.507 sind Adenosin- und Guanosinderivate mit ähnlichen Eigenschaften beschrieben. Die erfindungsgemässen Verbindungen unterscheiden sich strukturell signifikant von diesen vorbekannten Verbindungen, indem sie anstelle des Adenin- bzw. Guaninrestes einen in 2-Stellung durch Methyl, Aethyl oder Isopropyl, in 4-Stellung durch Carbamoyl und in 5-Stellung durch Nitrocarbamoyl substituierten Imidazolrest aufweisen.

Erfindungsgemäss lassen sich die Ribofuranosyl-imidazolderivate der obigen Formel I dadurch herstellen, dass man eine Verbindung der allgemeinen Formel

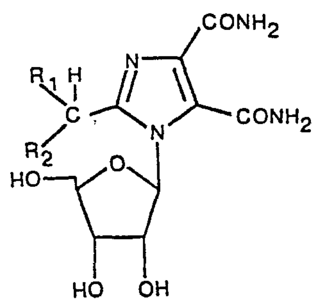

II

worin $R_1$ und $R_2$ die obige Bedeutung besitzen, nitriert.

Die Nitrierung der Verbindungen der Formel II wird nach an sich bekannten Methoden durchgeführt. So kann die Nitrierung beispielsweise mit Salpetersäure bewerkstelligt werden. Hydrolyse durch bei der Umsetzung gebildetes Wasser wird zweckmässig durch Zusatz eines wasserbindenden Mittels, wie konz. Schwefelsäure oder Oleum, unterdrückt. Die Nitrierung wird zweckmässig bei tiefen Temperaturen, vorzugsweise etwa im Bereich von —40° bis —10°C, durchgeführt.

Die Ausgansstoffe der Formel II sind ebenfalls neu und können in Analogie zur Herstellung bekannter Verbindungen hergestellt werden. Eine Herstellungsverfahren ist im nachfolgenden Schema I skizziert. Bezüglich der genauen Reaktionsbedingungen wird auf den Beispielteil verwiesen.

Schema I

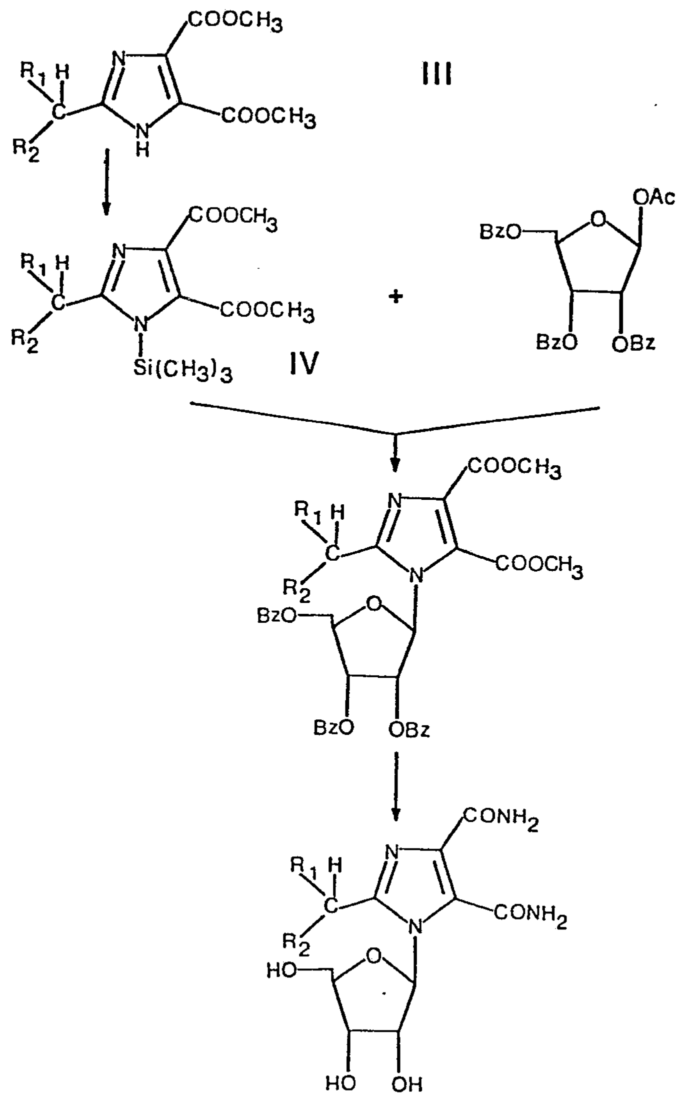

III

IV

V

VI

II

Im obigen Schema I bedeuten Bz die Benzoylgruppe und Ac die Acetylgruppe und besitzen $R_1$ und $R_2$ die obige Bedeutung.

Die Verbindungen der obigen Formeln IV und VI sind ebenfalls neu.

Die Verbindungen der Formel III sind aus Chem. Ber., *107,* 892 (1974) bekannt oder können in Analogie zur Herstellung der bekannten Verbindungen hergestellt werden. Die Verbindung der Formel V ist aus Helv. Chim. Acta, *47,* 1171 (1959) bekannt.

Die erfindungsgemässen Verbindungen besitzen wertvolle cardiale (wie coronardilatierende) und kreislaufdynamische Eigenschaften und können somit als Heilmittel, u.a. zur Behandlung von Angina pectoris Verwendung finden. Als Dosierungsrichtlinie kann eine Menge von 0,01—30 mg/kg Körpergewicht pro Tag gelten und sowohl als einmalige Dosis als auch — was bevorzugt ist — in mehreren Teildosen verabreicht werden.

Die erfindungsgemässen Verbindungen können als pharmazeutische Präparate mit direkter oder verzögerter Freigabe des Wirkstoffs in Mischung mit einem für die enterale, perkutane oder parenterale Applikation geeigneten organischen oder anorganischen inerten Trägermaterial, wie Wasser, Gelatine, Gummi arabicum, Milchzucker, Stärke, Magnesiumstearat, Talk, pflanzlichen Oelen, Polyalkylenglykolen, und Vaseline verwendet werden. Die pharmazeutischen Präparate können in fester Form, z.B. als Tabletten, Dragées, Suppositorien, Kapseln; in halbfester Form, z.B. als Salben; oder in flüssiger Form, z.B. als Lösungen, Suspensionen oder Emulsionen, vorliegen. Gegebenenfalls sind sie sterilisiert und bzw. oder enthalten weitere Hilfstoffe, wie Konservierungs-, Stabilisierungs-, Netz- oder Emulgiermittel, Mittel zur geschmacklichen Verbesserung, Salze zur Veränderung des osmotischen Druckes oder Puffersubstanzen. Die Herstellung der pharmazeutischen Präparate kann in der dem Fachmann geläufigen Weise erfolgen.

Die coronardilatierende Wirkung kann nach folgender Methode gemessen werden:

Für die Untersuchungen werden Bastardhunde im Gewicht zwischen 20 und 35 kg verwendet. Die Versuchstiere werden mit 35 mg/kg i.v. Pentobarbital narkotisiert, tracheal intubiert und mit Raumluft künstlich beatmet. Nach Oeffung des Thorax wird das Herz freigelegt und um den Ramus circumflexus der linken Coronararterie eine vorher geeichte Flowprobe eines elektromagnetischen Flowmeters zur Messung der durchfliessenden Blutmenge gelegt. Der arterielle Blutdruck wird über einen Katheter in der Aorta ascendens mit Drucktransducer gemessen. Die Pulswelle triggert einen Tachographen zur Messung der Herzfrequenz. Der Nachweis einer nitroglycerinähnlichen Wirkungsweise wurde mittels der coronaren Segmentanalyse nach Winbury erbracht (Winbury, M. et al., J. Pharmacol. Exp. Ther. 168 (1), 70—95, 1969).

Die Verbindungen werden, in Propylenglykol gelöst, intravenös appliziert. Die maximale Wirkung einer Substanz wird nach jeder Dosierung in Prozent des Ausgangswertes berechnet und die jeweilige Wirkungsdauer bestimmt.

In der folgenden Tabelle sind pharmakologische Daten von einigen erfindungsgemässen Verbindung zusammengestellt.

TABELLE

| Verbindung der Formel I | n | Dosis i.v. [mg/kg] | BP | | HR | | CABF | | $P_L$ | |
|---|---|---|---|---|---|---|---|---|---|---|
| | | | Δ % | [min] | Δ % | [mi-] | Δ % | [min] | Δ % | [min] |
| mit $R_1$ und $R_2$ = H | 5 | 3 | -55 | >60 | +8 | 20 | -30 | >60 | -38 | 60 |
| mit $R_1$ = $CH_3$; $R_2$ = H | 3 | 1 | -32 | >60 | -6 | >60 | -19 | >60 | -24 | 60 |
| mit $R_1$ und $R_2$ = $CH_3$ | 2 | 10 | -65 | 120 | +5 | 120 | -32 | 120 | -29 | 60 |

n = Anzahl der Tiere

BP = arterieller Blutdruck

HR = Herzfrequenz

CABF = Coronarfluss

$P_L$ = Druckgradient (Aortadruck minus peripherem Coronardruck)

Beispiel 1

15,0 g 2-Methyl-1-$\beta$-D-ribofuranosylimidazol-4,5-dicarboxamid werden bei —40°C in 120 ml rauchender Salpetersäure (D=1,50) gelöst. Dazu wird eine auf —20°C vorgekühlte Mischung aus 50 ml Oleum und 50 ml Nitromethan so zugetropft, dass die Reaktionstemperatur —30°C nicht übersteigt. Das Reaktionsgemisch wird danach 45 Minuten bei —30 bis —25°C gerührt und dann unter starkem Rühren auf 1 l eines Eis-Wasser-Gemisches getropft. Das ausgefallene Produkt wird abfiltriert, mit kaltem Wasser gewaschen und im Exsikkator über Kaliumhydroxid getrocknet. Nach Umkristallisation aus Aethanol erhält man 12,0 g 2-Methyl-$N^5$-nitro-1-(2,3,5-tri-O-nitro-$\beta$-D-ribofuranosyl)imidazol-4,5-dicarboxamid, Smp. 164°C (Zersetzung). Die Struktur der genannten Verbindung wurde durch eine Röntgenstrukturanalyse bestätigt.

In analoger Weise wurden die folgenden Verbindungen durch Nitrierung hergestellt:

2 - Aethyl - $N^5$ - nitro - 1 - (2,3,5 - tri - O - nitro - $\beta$ - D - ribofuranosyl)imidazol - 4,5 - dicarboxamid, Smp. 126—127°C (Zersetzung);

2 - Isopropyl - $N^5$ - nitro - 1 - (2,3,5 - tri - O - nitro - $\beta$ - D - ribofuranosyl)imidazol - 4,5 - dicarboxamid, Smp. 100—102°C.

Für die beiden obengenannten Verbindungen wurden keine Röntgenstrukturanalysen zur Bestätigung der Struktur durchgeführt.

Die als Ausgangsstoffe verwendeten Nukleoside können wie folgt hergestellt werden:

a) 19,8 g 2-methylimidazol-4,5-dicarbonsäure-dimethylester werden in 120 ml Benzol gelöst, mit 16,7 ml Triäthylamin und danach mit 18 ml Trifluormethansulfonsäure-trimethylsilylester bei 5°C versetzt und 3 Stunden bei Raumtemperatur gerührt. Dann werden die Phasen getrennt, und der Benzolextrakt zur Trockene eingedampft. Der so erhaltene, rohe 2-Methyl-1-(trimethylsilyl)imidazol-4,5-dicarbonsäure-dimethylester und 50,5 g 1-O-Acetyl-2,3,5-tri-O-benzoyl-$\beta$-D-ribofuranose werden in 500 ml Aethylenchlorid gelöst. Dazu werden 18 ml Trifluormethansulfonsäure-trimethylsilylester unter Argon getropft, und das Reaktionsgemisch wird 18 Stunden bei Raumtemperatur gerührt. Die Lösung wird unter Rühren auf 500 ml gesättigten Natriumhydrogencarbonatlösung gegossen, und das Reaktionsgemisch wird eine Stunde weitergerührt. Danach werden die Phasen getrennt, und die wässerige Phase mit 250 ml Methylenchlorid extrahiert. Die organische Phase wird über Natriumsulfat getrocknet und eingedampft. Der Rückstand wird über Kieselgel mit einem Gemisch von Hexan und Essigester chromatographiert. Nach Umkristallisation aus Aethanol erhält man 30,8 g 2-Methyl-1-(2,3,5-tri-O-benzoyl-$\beta$-D-ribofuranosyl)imidazol-4,5-dicarbonsäure-dimethylester, Smp. 121—122°C.

In analoger Weise wurden die folgenden Verbindungen durch Umsetzung von 1-O-Acetyl-2,3,5-tri-O-benzoyl-$\beta$-D-ribofuranose mit den silylierten 2-Aethyl- bzw. 2-Isopropylimidazol-4,5-dicarbonsäure-dimethylestern hergestellt:

2 - Aethyl - 1 - (2,3,5 - tri - O - benzoyl - $\beta$ - D - ribofuranosyl)imidazol - 4,5 - dicarbonsäure - dimethylester, Smp. 147°C;

2 - Isopropyl - 1 - (2,3,5 - tri - O - benzoyl - $\beta$ - D - ribofuranosyl)imidazol - 4,5 - dicarbonsäure - dimethylester, Smp. 147—148°C.

Die in der obigen Stufe als Ausgangsstoffe verwendeten Imidazol-4,5-dicarbonsäureester können wie folgt gewonnen werden:

Trockenes Chlorwasserstoffgas wird unter Rückfluss solange in eine Suspension von 14,6 g 2-Aethylimidazol-4,5-dicarbonsäure in 150 ml Methanol eingeleitet, bis kein Ausgangsmaterial mehr vorhanden ist (DC—Kontrolle). Danach wird die erhaltene Lösung bis zur Trockene eingedampft, und der Rückstand zweimal mit je 100 ml Methanol wieder eingedampft. Der Rückstand wird in 250 ml Eiswasser gelöst, mit einer gesättigten Natriumhydrogencarbonatlösung neutralisiert, und dreimal mit je 250 ml Essigester extrahiert. Die Essigesterextrakte werden über Natriumsulfat getrocknet und bis zur Trockene eingedampft. Der Rückstand wird aus Benzol umkristallisiert, wobei man 9,6 g 2-Aethylimidazol-4,5-dicarbonsäure-dimethylester erhält, Smp. 111°C.

In analoger Weise wird 2-Isopropylimidazol-4,5-dicarbonsäure-dimethylester, Smp. 150°C, durch Umsetzung der 2-Isopropylimidazol-4,5-dicarbonsäure mit Methanol hergestellt.

b) Eine Lösung von 26,0 g 2-Methyl-1-(2,3,5-tri-O-benzoyl-$\beta$-D-ribofuranosyl)imidazol-4,5-dicarbonsäure-dimethylester in 500 ml Methanol, das bei 0°C mit wasserfreiem Ammoniak gesättigt worden ist, wird 7 Tage in einem verschlossenen Druckkolben bei Raumtemperatur stehengelassen. Dann wird das Reaktionsgemisch bis zur Trockene eingedampft, und der Rückstand aus Methanol umkristallisiert. Man erhält 9,5 g 2-Methyl-1-$\beta$-D-ribofuranosylimidazol-4,5-dicarboxamid, Smp. 167°C.

In analoger Weise wurden die folgenden Verbindungen durch Ammonolyse hergestellt:

2-Aethyl-1-$\beta$-D-ribofuranosylimidazol-4,5-dicarboxamid, $[\alpha]_D^{25} = +59,4°$ (c=1,00, Dimethylsulfoxid);

2-Isopropyl-1-$\beta$-D-ribofuranosylimidazol-4,5-dicarboxamid, $[\alpha]_D^{25} = +43,3°$ (c = 1,00 Dimethylsulfoxid).

Beispiel 2

Herstellung von Kapseln nachstehender Zusammensetzung:

| | |
|---|---|
| Wirkstoff der Formel I | 250,0 mg |
| Milchzucker | 155,0 mg |
| Maisstärke | 30,0 mg |
| Talcum | 15,0 mg |
| | 450,0 mg |

Der Wirkstoff wird mit dem Milchzucker und der Maisstärke homogen vermischt, durch eine Siebmaschine passiert und nach Untermischen des Talcums in Gelatinekapseln abgefüllt.

| | |
|---|---|
| Kapselfüllgewicht | 450 mg |
| Wirkstoffgehalt | 250 mg |

Beispiel 3

Herstellung von Tabletten nachstehender Zussamensetzung:

| | |
|---|---|
| Wirkstoff der Formel I | 250,0 mg |
| Milchzucker | 100,0 mg |
| Maisstärke | 85,0 mg |
| Aethylcellulose | 10,0 mg |
| Talcum | 4,5 mg |
| Magnesiumstearat | 0,5 mg |
| | 450,0 mg |

Der Wirkstoff wird mit dem Milchzucker und der Maisstärke vermischt und mit einer Lösung der Aethylcellulose in 40 ml Methylenchlorid granuliert. Das Granulat wird bei 40°C getrocknet, mit dem Talcum und Magnesiumstearat vermischt und zu Tabletten gepresst.

| | |
|---|---|
| Gewicht einer Tablette | 450 mg |
| Wirkstoffgehalt einer Tablette | 250 mg |

**Patentansprüche**

1. Ribofuranosyl-imidazolderivate der allgemeinen Formel

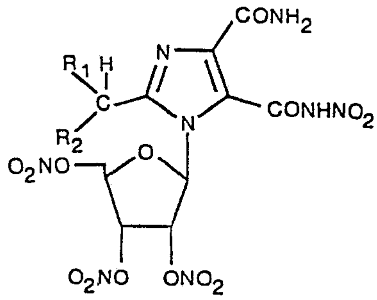

I

worin $R_1$ und $R_2$ je Wasserstoff oder Methyl bedeuten.

2. Verbindung gemäss Anspruch 1, worin $R_1$ und $R_2$ je Wasserstoff bedeuten.

3. Ribofuranosyl-imidazolderivate gemäss einem der Ansprüche 1 und 2 als pharmazeutische Wirkstoffe.

4. Ribofuranosyl-imidazolderivate gemäss einem der Ansprüche 1 und 2 als cardiovaskuläre Wirkstoffe.

5. Verfahren zur Herstellung von Ribofuranosylimidazolderivaten gemäß Anspruch 1, dadurch gekennzeichnet, dass man eine Verbindung der allgemeinen Formel

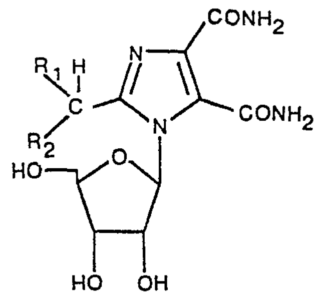

II

worin $R_1$ und $R_2$ die obige Bedeutung besitzen, nach an sich bekannten Methoden nitriert.

6. Arzneimittel enthaltend ein Ribofuranosyl-imidazolderivat gemäß. Anspruch 1.

7. Cardiovaskuläres Mittel enthaltend ein Ribofuranosyl-imidazolderivat gemäß Anspruch 1.

**Claims**

1. Ribofuranosyl-imidazole derivatives of the general formula

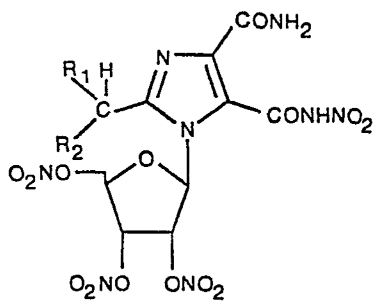

I

wherein $R_1$ and $R_2$ each signify hydrogen or methyl.

2. A compound in accordance with claim. 1, wherein $R_1$ and $R_2$ each signify hydrogen.

3. Ribofuranosyl-imidazole derivatives in accordance with one of claims 1 and 2 as pharmaceutically active substances.

4. Ribofuranosyl-imidazole derivatives in accordance with one of claims 1 and 2 as cardiovascular active substances.

5. A process for the manufacture of ribofuranosyl-imidazole derivatives in accordance with claim 1, characterized by nitrating a compound of the general formula

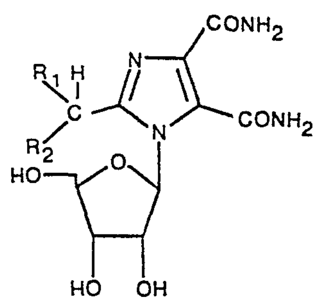

II

wherein $R_1$ and $R_2$ have the above significance, according to methods known per se.

6. A medicament containing a ribofuranosyl-imidazole derivative in accordance with claim 1.

7. A cardiovascular medicament containing a ribofuranosyl-imidazole derivative in accordance with claim 1.

**Revendications**

1. Dérivés de ribofuranosyl-imidazole de formule générale:

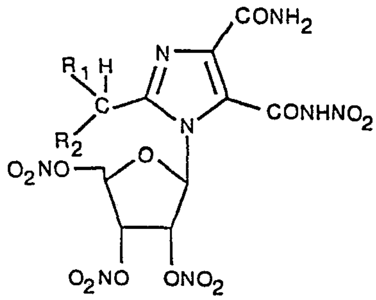

I

où $R_1$ et $R_2$ représentent chacun un hydrogène ou un méthyle.

2. Composé selon la revendication 1, où $R_1$ et $R_2$ représentent chacun un hydrogène.

3. Dérivés de ribofuranosyl-imidazole selon l'une des revendications 1 et 2 comme substances actives pharmaceutiques.

4. Dérivés de ribofuranosyl-imidazole selon l'une des revendications 1 et 2 comme substances actives cardiovasculaires.

5. Procédé de préparation de dérivés de ribofuranosyl-imidazole selon la revendication 1, caractérisé en ce qu'on nitre par des procédés classique un composé de formule générale:

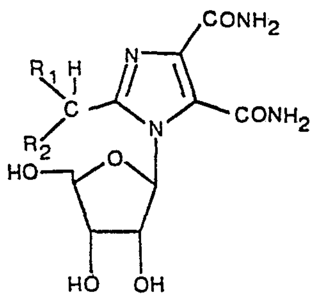

II

où $R_1$ et $R_2$ ont la signification ci-dessus.

6. Médicament contenant un dérivé de ribofuranosyl-imidazole selon la revendication 1.

7. Agent cardiovasculaire contenant un dérivé de ribofuranosyl-imidazole selon la revendication 1.